# EUROPÄISCHE PATENTSCHRIFT

(11) **EP 1 024 127 B1**
(45) Veröffentlichungstag und Bekanntmachung des Hinweises auf die Patenterteilung: **14.08.2002**
(21) Anmeldenummer: 00100058.7
(22) Anmeldetag: 05.01.2000
(51) Int. Cl.: C07C 29/80, C07C 31/22

(54) **Verfahren zur Herstellung von hochreinem Phytantriol**
Process for the preparation of highly pure phytantriol
Procédé pour la préparation de phytantriol de haute pureté

(30) Priorität: 28.01.1999 DE 19903459
(43) Veröffentlichungstag der Anmeldung: 02.08.2000
(73) Patentinhaber: BASF AKTIENGESELLSCHAFT, 67056 Ludwigshafen (DE)
(72) Erfinder: Streicher, Harald, Dr., 67069 Ludwigshafen (DE); Burst, Wolfram, 68199 Mannheim (DE); Däuwel, Jürgen, 67363 Lustadt (DE); Koppenhöfer, Jürgen, 67149 Meckenheim (DE)

(56) Entgegenhaltungen:
- DE-A- 19 524 928
- DE-B- 1 149 700
- PATENT ABSTRACTS OF JAPAN vol. 011, no. 080 (C-409), 11. März 1987 (1987-03-11) & JP 61 236737 A (KURARAY CO LTD), 22. Oktober 1986 (1986-10-22)

## Beschreibung

Die Erfindung betrifft ein Verfahren zur Herstellung und destillativen Feinreinigung von dem als Zusatz zu Haut- und Haarpflegemitteln begehrten 3,7,11,15-Tetramethyl-1,2,3-trihydroxyhexadecan (Phytantriol) der Formel I

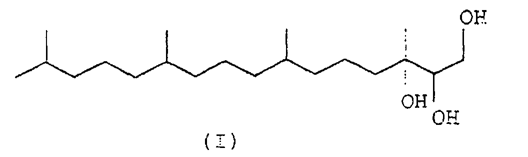

Phytantriol ist in der Kosmetikindustrie als Feuchthaltemittel und als Penetrationsverstärker für kosmetische Wirkstoffe, wie Panthenol, Vitamin A und Vitamin E (vgl. beispielsweise DE 42 15 501, EP 686 386, EP 679 383, DE 42 34 744, JP 04069316, JP 61236737, DE 14 67 925 und DE 11 42 428) begehrt.

Für die Herstellung dieses Wirkstoffs sind im wesentlichen zwei techmische Verfahren bekannt.
1) Aus DE 1 149 700 ist ein Verfahren zur Herstellung von dreiwertigen Alkoholen der allgemeinen Formel II 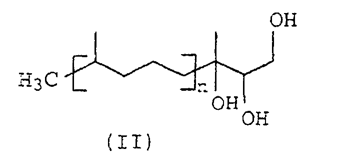 bekannt, bei dem man aus tertiären Alkoholen der allgemeinen Formel III 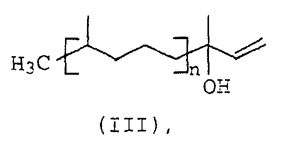 wie dem Isophytol (n = 3), durch Umsetzen in an sich bekannter Weise mit einer organischen Persäure, insbesondere mit Perameisensäure, und anschließende alkalische Verseifung Phytantriol (I) herstellen kann.
   Nachteilig an diesem Verfahren ist, daß die erhaltenen, bei hohen Temperaturen leicht zersetzlichen dreiwertigen Alkohole stark durch Nebenprodukte verunreinigt sind, die annähernd gleich hohe Siedepunkte aufweisen und daher selbst bei destillativer Auftrennung im Hochvakuumbereich (ca. 10⁻¹ bis 10⁻⁵ mbar), wie über Kurzwegdestillationen oder Molekulardestillationen, nicht in der für kosmetische Zwecke erwünschten Reinheit hergestellt werden können. Abgesehen davon, daß für solche Trennungen im Hochvakuumbereich sowohl sehr hohe Investitionskosten als auch hohe laufende Betriebskosten anfallen, sind bei solchen Gemischen mit geringen relativen Flüchtigkeiten mit vertretbarem Aufwand nur ungenügend reine Produkte erhältlich. Hohe Reinheiten erzielt man nur mit extrem hohem apparativem Aufwand und extrem niedrigen Destillationsausbeuten.
2) Aus der japanischen Patentveröffentlichung JP 61236737 A2 ist ein Verfahren zur Herstellung von Phytantriol bekannt, bei dem dieses in etwas größerer Reinheit anfällt. Bei diesem Verfahren wird Isophytol in Gegenwart von Vanadium- oder Molybdän-Verbindungen mit tert.-Butyl-hydroperoxid [(CH₃ )₃ C-OOH] umgesetzt und in den so erhaltenen Epoxiverbindungen anschließend mit Hilfe von sauren Katalysatoren eine Öffnung der Epoxidringe vorgenommen. Das so erhaltene Rohphytantriol wird dann durch Molekulardestillation bei 0,22 mbar gereinigt.

Nachteilig an diesem Verfahren ist die Verwendung von Schwermetallen, die in der Kosmetikindustrie unerwünscht ist, die relativ aufwendige Verfahrensführung sowie die Notwendigkeit einer Reinigung mittels Molekulardestillation.

Es war daher die Aufgabe der Erfindung, das als Wirkstoff in der Kosmetikindustrie begehrte Phytantriol in industriellem Maßstab auf einfache Weise, ohne Mitverwendung von Schwermetallverbindungen und ohne die Notwendigkeit einer Reinigung im Hochvakuum in sehr reiner Form herzustellen.

Es wurde nun überraschender Weise gefunden, daß man verunreinigtes Phytantriol, insbesondere das nach dem Verfahren von DE 1 149 700 auf relativ einfache Weise herstellbare, aber mit niedriger und/oder höher siedenden färbenden Nebenkomponenten verunreinigte Phytantriol auch ohne kostenaufwendige Molekulardestillation in sehr reiner Form und hoher Destillationsausbeute erhalten kann, wenn man sie einer sehr speziellen Rektifikation im Feinvakuum (d.h. bei einem Kopfvakuum von 0,1 bis 2 mbar) unterwirft.

Aus DE 19524928 war zwar ein Verfahren zur Gewinnung reiner Substanzen aus Gemischen hochsiedender luft- und/oder temperaturempfindlicher Substanzen, die eine hohe Trennleistung erfordern, durch Rektifikation unter speziellen Bedingungen im Feinvakuum bekannt, dennoch war es sehr überraschend, daß auch das hochsiedende und gegen thermische Belastungen extrem empfindliche Phytantriol im Feinvakuum mit Destillationsausbeuten von bis zu 95 % der Theorie zu hochreinem Phytantriol (Reinheit > 99 %) rektifiziert werden kann. Dies gilt insbesondere, da noch der UIC-News der Norbert Kukla UIC GmbH vom 07.12.1998 zu entnehmen ist, daß selbst einer der bedeutenden Hersteller von Phytantriol trotz intensiver Forschung über die Hochreinigung von Phytantriol nur die Kurzwegdestillation, d.h., eine sehr aufwendige Destillation unter extrem hohem Vakuum, für geeignet hält, obwohl die Forschung - nachweislich der Angaben in der genannten UIC-News - neben der Erfüllung höherer Qualitätsansprüche auch die verständliche Forderung nach Produktivitätssteigerung und nach Reduzierung der Kosten zum Ziel hatte.

Gegenstand der Erfindung ist dementsprechend ein Verfahren zur Herstellung von hochreinem Phytantriol, das dadurch gekennzeichnet ist, daß man mit niedriger und/oder höher siedenden färbenden Nebenprodukten verunreinigtes Phytantriol im Feinvakuum in Kolonnen enthaltend Metallgewebepackungen mit geordneter Struktur rektifiziert, wobei man
a) die Flüssigkeitsverteilung mit Kanalverteilern mit gleich oder mehr als 500 Abtropfstellen pro m² vornimmt,
b) die Kanäle der Flüssigkeitsverteiler im Winkel von etwa 90° zu den Gewebelagen der direkt unter diesen Verteilern befindlichen Packungselemente anordnet,
c) unterhalb der Flüssigkeitsverteiler 2 oder mehrere Packungselemente mit einer Höhe von nur 20 bis 100 mm Höhe einsetzt, deren Gewebelagen jeweils um 90° zueinander gedreht sind,
d) unter absolutem Luftausschluß arbeitet und
e) daß man die Rektifikation streng adiabatisch betreibt.

Besonders vorteilhaft gestaltet sich die Herstellung von hochreinen Phytantriol, wenn man als mit Nebenprodukten verunreinigtes Phytantriol ein Phytantriol verwendet, welches durch Umsetzung von Isophytol mit Perameisensäure und anschließende Verseifung des gebildeten Reaktionsproduktes mit alkalischen Mitteln nach dem aus DE 1 149 700 bekannten Verfahren erhalten wurde.

Vermutlich lagert sich bei der Umsetzung von Isophytol mit Perameisensäure in einer ersten Reaktion das als Ausgangsverbindung eingesetzte Isophytol unter Allylumlagerung in das Phytolformiat um, in welchem anschließend unter Einwirkung der Perameisensäure die so entstandene β,γ-Doppelbindung epoxidiert wird. Aus dem erhaltenen 1-Formyloxy-2,3-epoxy-phytol bildet sich schließlich das 1,3-Diformyloxy-2-hydroxy-3,7,11,15-tetramethyl-hexadecan, aus dem durch alkalische Verseifung das 1,2,3-Trihydroxy-3,7,11,15-tetramethyl hexadecan gebildet wird. Hierbei ist es besonders zweckmäßig, die Allylumlagerung unter Bildung des Phytylformiats vollständig durchzuführen, bevor die Epoxidierung mit Perameisensäure vorgenommen wird. Dies kann mit Vorteil so erfolgen, daß man das Isophytol zuerst mit Ameisensäure allein umsetzt und erst nach vollständiger Allylumlagerung die Epoxidierung durch Zugabe von Wasserstoffperoxid, welches mit der Ameisensäure in situ Perameisensäure bildet, erfolgen läßt.

Die Umsetzung mit Perameisensäure, bzw. mit Ameisensäure und dem Wasserstoffperoxid erfolgt im allgemeinen bei Raumtemperatur oder leicht erhöhter Temperatur, sodaß als Reaktionstemperaturen Temperaturen von 20 bis 60 °C, vorzugsweise 30 bis 50 °C, genannt werden können.

Die Verseifung der Trihydroxy-diformiat-Verbindung in das Phytantriol erfolgt mit alkalischen Mitteln. Hierfür könnte z.B. verdünnte Natronlauge oder Kalilauge verwendet werden.

Man erhält das rohe Phytantriol als zähflüssiges Öl, welches erfindungsgemäß durch Rektifikation im Feinvakuum in sehr reiner Form (in Reinheiten größer als 99 %) erhalten werden kann.

Für die Auftrennung von hochsiedenden Substanzgemischen, die eine hohe Trennleistung erfordern, d.h. deren Siedepunkte eng beieinander liegen, verwendet man im allgemeinen Rektifizierkolonnen, die in regelmäßiger Geometrie systematisch aufgebaute Packungen mit definierten Durchtrittsbereichen für die Gegenstromphasen aufweisen, da sich regelmäßig strukturierte Packungen gegenüber Füllkörpern durch höhere Belastbarkeit und eine bessere Trennwirkung auszeichnen, einen geringeren spezifischen Druckverlust sowie ein geringeres erforderliches Packungsvolumen und daher auch eine kleinere notwendige Stoff- und Wärme-austauschende Höhe aufweisen. Sie werden daher bei allen Vakuumrektifikationen eingesetzt, bei denen es wegen der Temperaturempfindlichkeit des zu trennenden Gemisches besonders auf eine Begrenzung des Kolonnendruckverlustes ankommt. Besonders geeignete Kolonnenpackungen sind Metallgewebepackungen vom Typ BX und CY der Firma Sulzer (vgl. Firmensohrift "Trennkolonnen für Destillation und Absorption" der Firma Sulzer) und ähnlich wirksame Metallgewebepackungen anderer Firmen, wie der Montz GmbH.

Eine schematische Darstellung solcher Kolonnen findet man beispielsweise auf Seite 103 des Lehrbuches "Thermische Trennverfahren" von Klaus Sattler, VCH Verlagsges.mbH, Weinheim (BRD), 1988. Bezüglich näherer Einzelheiten über die Rektifikation von Substanzgemischen verweisen wir auf dieses Lehrbuch von Klaus Sattler, Seiten 101-225, insbesondere 120-160 und 199-214.

Bei Betriebskolonnen sind Kopfdrucke von 0,5 mbar gerade noch mit vertretbarem technischem Aufwand realisierbar. Die höchste Produkttemperatur tritt im Sumpf einer Kolonne auf. Sie wird außer vom Kopfdruck maßgeblich von dem für die erforderliche Trennleistung bedingten Druckverlust der Kolonneneinbauten bestimmt.

Für zahlreiche hochsiedende Gemische ist die thermische Belastbarkeit so gering, daß sich trotz Verwendung der beschriebenen Metallgewebepackungen mit geordneter Struktur und Kopfdrucken in der Kolonne von nur 0,5 bis 1 mbar durch den Druckverlust an den für die erforderliche Trennleistung benötigten Gewebepackungen im Sumpf Temperaturen auftreten würden, die oberhalb des Zersetzungsbereichs der zu trennenden Verbindungen liegt. Für die destillative Auftrennung solcher Gemische arbeitet man daher bisher im allgemeinen im Hochvakuumbereich (ca. 10⁻¹ bis 10⁻⁵ mbar), d.h. man wendet Kurzwegdestillationen oder Molekulardestillationen an. Bei Gemischen mit geringen relativen Flüchtigkeiten sind mit diesen Destillationen hohe Reinheiten jedoch nur mit niedrigen Destillationsausbeuten erzielbar.

Bei Betriebskolonnen sind Kopfdrucke von 0,5 bis 1 mbar noch mit vertretbarem technischem Aufwand realisierbar. Phytantriol weist bei einem Druck von 1 mbar eine Siedetemperatur von ca. 200°C auf. Durch die begrenzte thermische Stabilität von Phytantriol ist die Temperatur im Kolonnensumpf auf ca. 210 bis 230°C begrenzt. Eine Rektifikationskolonne darf daher maximal mit einem Sumpfdruck von nur 3 mbar betrieben werden. Dies bedeutet, daß nur ein Druckverlust von ca. 2 bis 2,5 mbar zwischen Kopf und Sumpf der Kolonne toleriert werden kann. Dies ist sehr schwierig zu erreichen, da zur Feinreinigung von Phytantriol eine Trennleistung von etwa 10 bis 30 Trennstufen erforderlich ist und man normalerweise mit einem Druckverlust von 0,3 bis 0,5 mbar pro Trennstufe rechnen muß.

Gemäß Merkmal a) des Hauptanspruch wird eine Flüssigkeitsverteilung mit Kanalverteilern mit gleich oder mehr als 500 Abtropfstellen beansprucht. Ähnliche auch "Kapillarverteiler" genannte aber runde Verteiler werden von den Firmen Sulzer und Montz vertrieben und werden beispielsweise in EP 512 277 beschrieben. Bekannte Kanalverteiler weisen im allgemeinen nur 50 bis 60 Abtropfstellen pro m² auf.

Die Verwendung der Kanalverteiler bewirkt bei der Rektifikation von Phytantriol auf 2 verschiedene Weisen eine Verminderung des sogenannten Druckverlustes. Zum einen bedingen sie eine schnelle äußerst feine Verteilung und damit letztlich eine bessere Nutzung der Packung zum Verteilen des zu trennenden Gemisches und zum anderen eine sehr geringe Berieselungsdichte. Für Sulzer-Packungen vom BX-Typ werden Flüssigkeitsbelastungen ab ca. 0,2 m³/m²·h als untere Belastungsgrenze angegeben. Durch die erfindungsgemäße Anwendung der Kanalverteiler mit gleich oder mehr als 500, vorzugsweise 900 bis 1200 Abtropfstellen werden beim Phytantriol Flüssigkeitsbelastungen beim erfindungsgemäßen Verfahren von nur 0,03 bis 0,3 m³/m²·h am Kopf und 0,03 bis 1,0 m³/m²·h im Abtriebsteil der Kolonne erreicht. Überraschenderweise wurde gefunden, daß auch bei so niedrigen Flüssigkeitsbelastungen, die für eine optimale Trennleistung erforderliche vollständige Benetzung der Metallpackungen sichergestellt ist. Durch diese geringe Berieselungsdichte wird die Gasbelastung in der Kolonne und damit der Druckverlust außerordentlich gering.

Zur Erreichung einer optimalen Trennleistung ist jedoch nicht nur eine hohe Abtropfstellenzahl sondern auch die Anordnung der Verteiler im Bezug auf die Packungselemente wichtig.

Eine Lage einer Gewebepackung besteht im allgemeinen aus einer Vielzahl üblicherweise einzelner, 170 mm hoher Gewebelagen. Jede Lage wird beim Einbau um jeweils 90° im Bezug auf die vorhergehende eingebaut. Die Anordnung der Verteiler erfolgt ebenfalls um 90° gedreht in Bezug auf das direkt unter den Verteilern befindliche Packungselement.

Die Flüssigkeit breitet sich nun auf einer dieser Gewebelagen in einem bestimmten Winkel aus. Nach einer von dem Ausbreitungswinkel und dem Abstand zweier Abtropfstellen abhängigen Einlauflänge hat sich ein gleichmäßiger Film über einer Gewebelage ausgebildet.

Eine optimale Ausnutzung der Packung, d.h. eine schnellstmögliche Verteilung der Flüssigkeit auf allen Gewebelagen erzielt man, wenn man an dieser Stelle die Packung um 90° dreht.

In dem erfindungsgemäßen Reinigungsverfahren werden daher unterhalb der Flüssigkeitsverteiler 2 oder mehrere Packungselemente mit einer Höhe von nur 20 bis 100 mm, vorzugsweise 25 bis 50 mm, insbesondere 35 bis 45 mm eingesetzt, deren Gewebelagen jeweils um 90° zueinander gedreht sind. Durch das Trennen der Packung in Elemente mit geringerer Höhe kann eine schnellstmögliche Verteilung und damit eine optimale Ausnutzung der Packung zum Trennen erzielt werden. Bei der herkömmlichen Packungsanordnung dagegen beträgt die Einlauflänge ca. 340 mm, was bedeutet, daß bei einer Packungshöhe von 2 Metern ca. % der Packung nicht voll für die eigentliche Trennoperation genutzt werden.

Gemäß Merkmal d) soll die Rektifikation unter absolutem Luftausschluß durchgeführt werden.

Laborversuche haben gezeigt, daß es bei Phytantriol bei den für die Rektifikation benötigten hohen Temperaturen schon bei geringsten Leckagen in der Destillationseinrichtung zu einer Dunkelfärbung des Produktes kommt, die Aufgrund der hohen Qualitätsanforderungen nicht toleriert werden kann. Die Verwendung von neu entwickelten besonders hochwertigen Dichtungsmaterialien, wie Helicoflex® der Firma Cefilac, zur Abdichtung von Flanschen und/oder Öffnungen für Geräte zur Prozeßüberwachung ist daher unbedingt notwendig. Zur Abdichtung von Flanschen verwendet man mit besonderem Vorteil sogenannte Schweißlippendichtungen, wie sie beispielsweise in den deutschen Patentschriften DE 27 10 859, DE 39 12 478 oder DD 44 07 728 beschrieben sind.

Wie bereits dargestellt, zirkulieren in den erfindungsgemäßen Feinvakuumrektifikationskolonnen nur geringe Stoffströme. Daher führt jeder Wärmeverlust sofort zu wilder, d.h. unkontrollierter Kondensation an der Kolonnenwand, welche die Trennleistung der Kolonne reduziert. Der streng adiabatische Betrieb der Kolonne kann am besten durch eine Kombination von Isolation und Schutzbeheizung der Kolonne gewährleistet werden.

Die technische Ausführung einer solchen Schutzbeheizung wird mit Vorteil praktisch auf folgende Weise realisiert: Auf einer ersten Isolierschicht auf dem Kolonnenmantel wird ein Blechmantel angebracht. Dieser Blechmantel wird erneut isoliert. Auf diese Iso-Herschicht wird dann ein weiterer Blechmantel und die Heizung aufgebracht und diese schließlich nach außen isoliert. Die Regelung der Heizung erfolgt dann derart, daß die Temperaturdifferenz zwischen dem Kolonnenmantel und dem ersten Blechmantel Null beträgt.

Für die erfindungsgemäße Feinreinigung von Phytantriol ist es notwendig, daß man bei der Rektifikation mit Kopfdrucken von 0,2 bis 1 mbar, vorzugsweise 0,5 bis 1 mbar und mit Sumpfdrucken von 1 bis 4 mbar, vorzugsweise 1,5 bis 3,5 mbar, insbesondere 2 bis 3 mbar, arbeitet.

Wegen der thermischen Labilität kann man die Rektifikation von Phytantriol gemäß dem Hauptanspruch, d.h. bei Verwendung der bekannten Gewebepackungen in Packungskolonnen von maximal 5 m Höhe durchführen.

Bei der erfindungsgemäßen Rektifikation von Phytantriol werden die Packungskolonnen mit einer Flüssigkeitsbelastung zwischen 0,03 und 0,3 m³/m²·h im Verstärkungsteil der Kolonne und mit einer Flüssigkeitsbelastung zwischen 0,03 und 1,0 m³/m²·h im Abtriebsteil der Kolonnen betrieben.

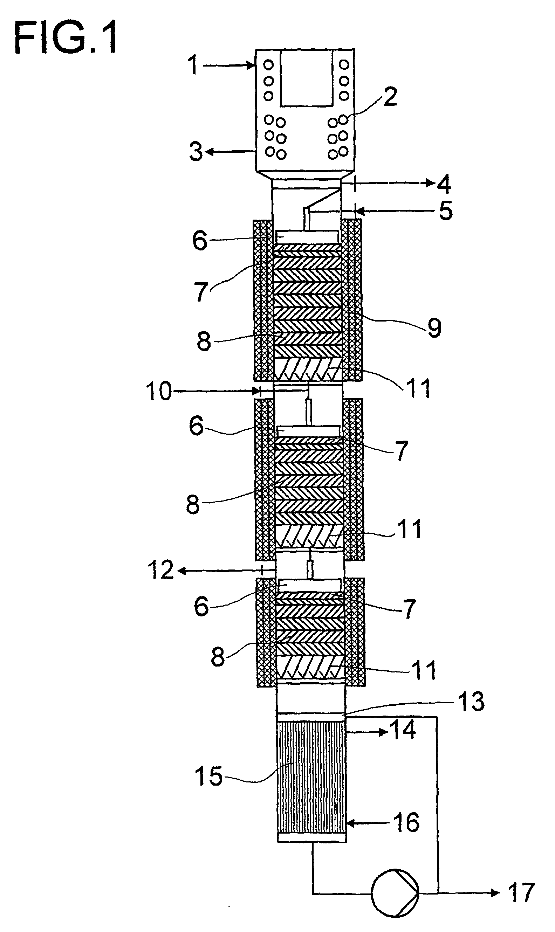

Eine für die Rektifikation von Phytantriol einsetzbare Packungskolonne ist in Figur 1 schematisch dargestellt.

Hierin bedeuten:
- 1: Zulauf für Kühlmedium
- 2: Kondensator
- 3: Ablauf für Kühlmedium
- 4: Destillat
- 5: Rücklauf
- 6: Kanal-Flüssigkeitsverteiler mit gleich oder mehr als 500 Abtropfstellen/m²
- 7: Packungselemente mit einer Höhe von 20 bis 100 mm
- 8: Packungselemente mit einer Höhe von ca. 170 mm
- 9: Schutzheizung
- 10: Zulauf für Roh-Phytantriol
- 11: Flüssigkeitssammler
- 12: Seitenabzug
- 13: Dichtungselement
- 14: Ablauf für Heizmedium (Ausgang)
- 15: Rieselfilmverdampfer
- 16: Zulauf für Heizmedium (Eingang)
- 17: Sumpfabzug.

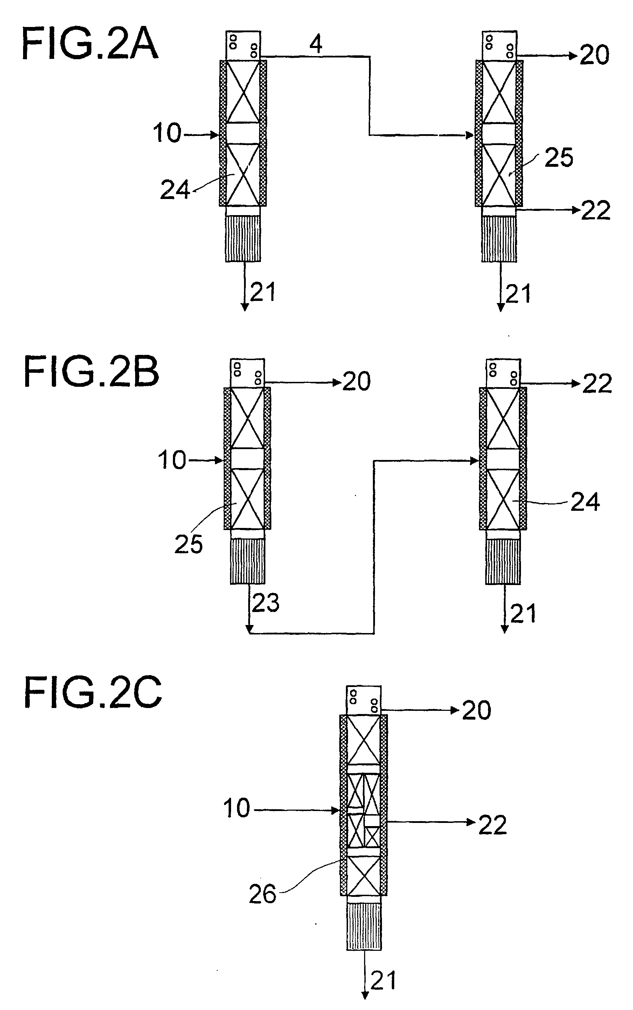

Zur Abtrennung von färbenden leichter siedenden Verunreinigungen und färbenden höher siedenden Verunreinigungen sind im allgemeinen und auch beim Phytantriol 2 Kolonnen notwendig. Die Figuren 2a und 2b stellen schematisch 2 mögliche Destillationskonzepte für die Rektifikation von Phytantriol in 2 Packungskolonnen dar. Hierin bedeuten
- 10: Zulauf für Phytantriol
- 20: Ablauf für Leichtsieder
- 21: Ablauf für Schwersieder
- 4: Destillat
- 22: Ablauf für Phytantriol rein, d.h. Reinheit > 99 %
- 23: Sumpf-Strom
- 24: Rektifikation zur Abtrennung der Schwersieder
- 25: Rektifikation zur Abtrennung der Leichtsieder

Man kann aber auch in nur einer Kolonne in zwei aufeinanderfolgenden Arbeitsgängen arbeiten, oder aber unter Verwendung einer, der Verwendung von zwei Kolonnen äquivalenten Destillationseinrichtung, wie einer sogenannten Trennblechkolonne arbeiten. Unter einer Trennblechkolonne versteht man allgemein eine Kombination von 2 getrennten Kolonnenpackungen innerhalb von einem äußeren Kolonnenmantel, an dem sich Zulauf und Seitenabzug befinden.

Eine solche Trennblechkolonne ist schematisch in Figur 2c dargestellt. Hierin haben 10, 20, 21 und 22 die gleiche Bedeutung wie in den Figuren 2a und 2b und 26 bedeutet Trennblechkolonne.

Mit Hilfe des erfindungsgemäßen Rektifikationsverfahrens ist es möglich, verunreinigtes Phytantriol insbesondere das bei der Umsetzung von Isophytol mit Perameisensäure und anschließende alkalische Verseifung erhaltene unreine Phytantriol auch im Feinvakuum, d.h. bei Drucken von 0,2 bis 2 mbar, vorzugsweise 0,5 bis 1 mbar in Kolonnen mit druckverlustarmen Gewebepackungen mit Destillationsausbeuten von bis zu 95 % zu rektifizieren.

Beispielhafte Beschreibung des Verfahrens zur Reinigung von Phytantriol mit Kolonnen gemäß Figur 1 nach dem Destillationskonzept gemäß Figur 2a.

Das gemäß dem in DE 1 149 700 beschriebene Verfahren hergestellte Phytantriol mit einem Gehalt von etwa 85 % wurde bei einer Temperatur zwischen 150 und 200°C durch den Zulauf 10 in die Mitte der ersten Rektifikationskolonne mit einer Packungshöhe von 3 bis 5 m vom Typ Sulzer BX oder Montz A3 eingetragen. Die Flüssigkeit wurde am Kopf und am Zulauf der Kolonne mit den neu entwickelten Hochleistungs-Kanalverteilern 6 gleichmäßig über den Kolonnenquerschnitt verteilt. Unterhalb der Kanalverteiler befanden sich die Packungselemente 7 mit einer Höhe von nur 20 bis 100 mm Höhe. Die Gewebelagen der direkt unter den Kanalverteilern 6 angeordneten Packungselemente waren zu den Kanälen der Flüssigkeitsverteiler im Winkel von 90° verdreht. Die Kolonne war mit einer Schutzheizung 9 ausgestattet und wurde adiabatisch betrieben. Flansche und Stutzen waren mit Schweißlippendichtungen bzw. hochwertigen Metalldichtungen versehen.

Der Kopfdruck der ersten Kolonne betrug ca. 0,5 bis 1 mbar. Das Rücklauf- verhältnis betrug 0,5 bis 2. Die in der Kolonne zurücklaufende Flüssigkeit hatte eine Temperatur zwischen 130 und 180°C. Am Sumpf der Kolonne wurden 5 bis 20 % des Zulaufstromes entnommen.

Das am Kopf der ersten Kolonne entnommene Produkt mit einer Temperatur von 150 bis 200°C wurde in die Mitte der zweiten Rektifikationskolonne mit 3 bis 5 m Packungshöhe vom Typ Sulzer BX oder Montz A3 gefördert. Die Flüssigkeit wurde am Kopf und am Zulauf mit den neu entwickelten Hochleistungs-Kanalverteilern über den Kolonnenquerschnitt verteilt. Auch diese Kolonne war mit einer Schutzheizung ausgerüstet und wurde adiabatisch betrieben. Flansche und Stutzen waren mit Schweißlippendichtungen bzw. hochwertigen Metalldichtungen versehen.

Der Kopfdruck der zweiten Kolonne betrug ca. 0,5 mbar. Das Rücklaufverhältnis betrug 2 bis 5. Die in die Kolonne zurücklaufende Flüssigkeit hatte eine Temperatur zwischen 130 bis 180°C. Am Kopf und im Sumpf der Kolonne wurden 5 bis 20 % des Zulaufstromes entnommen. Das im Seitenabzug unmittelbar oberhalb des Verdampfers der zweiten Kolonne entnommene Phytantriol war nahezu farblos (Farbzahl nach APHA kleiner als 10) und wies eine Reinheit von mehr als 99 % auf. Die Ausbeute an so reinem Produkt betrug bis zu 95 % der Theorie.

## Patentansprüche

1. Verfahren zur Herstellung von hochreinem Phytantriol, **dadurch gekennzeichnet, daß** man mit niedriger und/oder höhersiedenden Nebenprodukten verunreinigtes Phytantriol im Feinvakuum in Kolonnen enthaltend Metallgewebepackungen mit geordneter Struktur rektifiziert, wobei man
a) die Flüssigkeitsverteilung mit Kanalverteilern mit gleich oder mehr als 500 Abtropfstellen pro m² vornimmt,
b) die Kanäle der Flüssigkeitsverteiler im Winkel von etwa 90° zu den Gewebelagen der direkt unter diesen Verteilern befindlichen Packungselemente anordnet,
c) unterhalb der Flüssigkeitsverteiler 2 oder mehrere Pakkungselemente mit einer Höhe von nur 20 bis 100 mm Höhe einsetzt, deren Gewebelagen jeweils um 90° zueinander gedreht sind,
d) unter absolutem Luftausschluß arbeitet und
e) daß man die Rektifikation streng adiabatisch betreibt.

2. Verfahren gemäß Anspruch 1, **dadurch gekennzeichnet, daß** man als mit niedriger und/oder höher siedenden Nebenprodukten verunreinigtes Phytantriol ein Phytantriol verwendet, welches durch Umsetzen von Isophytol mit Perameisensäure und anschließende Verseifung des gebildeten Reaktionsproduktes mit alkalischen Mitteln in an sich bekannter Weise erhalten wurde.

3. Verfahren gemäß Anspruch 1, **dadurch gekennzeichnet, daß** die Flüssigkeitsverteilung gemäß a) mit Kanalverteilern mit 900 bis 1200 Abtropfstellen pro m² vornimmt.

4. Verfahren gemäß Anspruch 1, **dadurch gekennzeichnet, daß** man den absoluten Luftausschluß gemäß d) durch Verwendung besonders hochwertiger Dichtungsmaterialien oder durch den Einsatz von sogenannten Schweißlippendichtungen gewährleistet.

5. Verfahren gemäß Anspruch 1, **dadurch gekennzeichnet**₁ daß man den adiabatischen Betrieb der Kolonne gemäß e) durch eine Kombination aus Isolation und Schutzbeheizung der Kolonne gewährleistet.

6. Verfahren gemäß Anspruch 1, **dadurch gekennzeichnet, daß** man bei der Rektifikation von Phytantriol mit Kopfdrücken von 0,5 bis 1 mbar und mit Sumpfdrücken von 1 bis 4 mbar arbeitet.

7. Verfahren gemäß Anspruch 1, **dadurch gekennzeichnet, daß** bei der Rektifikation von Phytantriol die Kolonnen mit einer Flüssigkeitsbelastung zwischen 0,03 und 0,3 m³/m².h am Kopf und einer Flüssigkeitsbelastung zwischen 0,03 und 1,0 m³/m²·h im Abtriebsteil betrieben werden.

## Claims

1. A process for preparing high-purity phytantriol, which comprises rectifying phytantriol which is contaminated with lower and/or higher boiling byproducts under medium vacuum in columns containing metal packings with ordered structure, wherein
a) the liquid distribution is carried out by channel distributors with a minimum of 500 drip points per m²,
b) the channels of the liquid distributors are arranged at an angle of about 90° to the cloth layers of the packing elements located directly below these distributors,
c) 2 or more packing elements which have a height of only 20 to 100 mm and whose cloth layers are each rotated by 90° relative to one another are inserted underneath the liquid distributors,
d) air is absolutely excluded during operation, and
e) the rectification is carried out strictly adiabatically.

2. A process as claimed in claim 1, wherein the the phytantriol which is contaminated with lower and/or higher boiling byproducts is a phytantriol which has been obtained by reacting isophytol with performic acid and subsequently hydrolyzing the product formed in the reaction with alkaline agents in a manner known per se.

3. A process as claimed in claim 1, wherein the liquid distribution in a) is carried out with channel distributors with 900 to 1200 drip points per m² .

4. A process as claimed in claim 1, wherein the absolute exclusion of air in d) is ensured by using particularly high-quality sealing materials or employing so-called welded lip seals.

5. A process as claimed in claim 1, wherein the adiabatic operation of the column in e) is ensured by a combination of insulation and protective heating of the column.

6. A process as claimed in claim 1, wherein overhead pressures of from 0.5 to 1 mbar and bottom pressures of from 1 to 4 mbar are used in the rectification of phytantriol.

7. A process as claimed in claim 1, wherein the columns are operated with a liquid flow rate between 0.03 and 0.3 m³/m².h at the top and a liquid flow rate between 0.03 and 1.0 m³/m²·h in the stripping section during the rectification of phytantriol.

## Revendications

1. Procédé pour la préparation de phytantriol de haute pureté, **caractérisé par le fait qu'**on rectifie du phytantriol souillé par des sous-produits à point d'ébullition faible et/ou élevé, sous vide poussé, dans des colonnes contenant des garnissages de tissu métallique ayant une structure ordonnée, tandis qu'on
a) procède à la répartition des fluides avec des répartiteurs à canaux ayant 500 ou plus emplacements d'écoulement de goutte par m².
b) on dispose les canaux des répartiteurs de fluide sous un angle d'environ 90° par rapport aux couches de tissu des éléments de garnissage se trouvant directement en dessous de ces répartiteurs,
c) on dispose sous les répartiteurs de fluide 2 ou plus de 2 éléments de garnissage ayant une hauteur de seulement 20 à 100 mm, dont les couches de tissus sont à chaque fois tournées de 90° l'une par rapport à l'autre,
d) on opère en l'absence totale d'air et
e) on effectue la rectification dans des conditions strictement adiabatiques.

2. Procédé selon la revendication 1, **caractérisé par le fait qu'**on utilise comme phytantriol souillé par des sous-produits à point d'ébullition faible et/ou élevé, un phytantriol qui a été obtenu par réaction d'isophytol avec de l'acide performique et ensuite saponification du produit de réaction formé avec des agents alcalins, de manière connue en soi.

3. Procédé selon la revendication 1, **caractérisé par le fait qu'**on effectue la répartition de fluide selon a) avec des répartiteurs à canaux ayant 900 à 1200 emplacements d'égouttage par m².

4. Procédé selon la revendication 1, **caractérisé par le fait qu'**on assure l'absence totale d'air selon d) par utilisation de matériaux d'étanchéité de qualité particulièrement élevée ou par installation de joints à lèvres à soudure.

5. Procédé selon la revendication 1, **caractérisé par le fait qu'**on assure le fonctionnement adiabatique de la colonne selon e) par une combinaison d'isolement et de chauffage protecteur de la colonne.

6. Procédé selon la revendication 1, **caractérisé par le fait qu'**on opère lors de la rectification du phytantriol avec des pressions de tête de 0,5 à 1 mbar et avec des pressions de bas de colonne de 1 à 4 mbar.

7. Procédé selon la revendication 1, **caractérisé par le fait que** dans la rectification du phytantriol on fait fonctionner les colonnes avec une charge de fluide entre 0,03 et 0,3 m³/m².h en tête et avec une charge de fluide entre 0,03 et 1,0 m³/m².h dans la partie d'entraînement.
